(19) Europäisches Patentamt
European Patent Office
Office européen des brevets

(11) **EP 4 130 621 B1**

(12) **EUROPEAN PATENT SPECIFICATION**

(45) Date of publication and mention
of the grant of the patent:
**09.07.2025 Bulletin 2025/28**

(21) Application number: **21781640.4**

(22) Date of filing: **30.03.2021**

(51) International Patent Classification (IPC):
*G01N 33/02* (2006.01)     *G06N 20/00* (2019.01)
*F25D 11/00* (2006.01)

(52) Cooperative Patent Classification (CPC):
**F25D 11/003; F25D 29/003; G06N 20/00;**
F25D 2700/12; G01N 33/02

(86) International application number:
**PCT/JP2021/013482**

(87) International publication number:
**WO 2021/200915 (07.10.2021 Gazette 2021/40)**

(54) **PERISHABLE PRODUCT CONTAINING DEVICE, METHOD OF OPERATING THE PERISHABLE PRODUCT CONTAINING DEVICE AND COMPUTER PROGRAM**

SPEICHERVORRICHTUNG FÜR VERDERBLICHE PRODUKTE, VERFAHREN ZUM BETRIEB DER VORRICHTUNG UND COMPUTERPROGRAMM

DISPOSITIF DE STOCKAGE DE PRODUITS PÉRISSABLES, PROCÉDÉ DE FONCTIONNEMENT DU DISPOSITIF ET PROGRAMME INFORMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2020 JP 2020065240**

(43) Date of publication of application:
**08.02.2023 Bulletin 2023/06**

(73) Proprietor: **Daikin Industries, Ltd.**
**Osaka-shi, Osaka 530-0001 (JP)**

(72) Inventors:
• **TANNO, Shouichi**
**Osaka-shi, Osaka 530-8323 (JP)**
• **SATOU, Kiichirou**
**Osaka-shi, Osaka 530-8323 (JP)**
• **MATSUI, Hidenori**
**Osaka-shi, Osaka 530-8323 (JP)**

(74) Representative: **Hoffmann Eitle**
**Patent- und Rechtsanwälte PartmbB**
**Arabellastraße 30**
**81925 München (DE)**

(56) References cited:
WO-A1-2018/017450     WO-A1-2018/017817
CN-A- 110 657 836     JP-A- 2019 041 601
JP-A- 2019 041 601     US-A1- 2018 210 418
US-A1- 2019 390 897     US-A1- 2020 012 247
US-A1- 2020 097 776     US-B2- 10 908 582

## Description

Technical Field

The present disclosure relates to a perishable product containing device, a method of operating the perishable product containing device and a computer program comprising instructions to cause the perishable product containing device to carry out the steps of the method.

Background Art

JP 2019 41601 A discloses that the internal environment is controlled by inferring the amount of activated gas generated in time for the desired shipment timing and generating the activated gas based on the storage knowledge base, which is made by ruling of the optimum storage conditions based on the past storage experience and bringing thereof into knowledge, and environmental measurement information regarding gas metabolism amount. It is also described that software processing using regression analysis, which is a system of machine learning, is used to infer the amount of generated activated gas. A storage container, an adjustment unit and an environmental information acquisition unit are known from WO 2018/017450 A1 and WO 2018/017817 A1.

US 2020/012247 A1 relates to an artificial intelligence device mounted on a wine refrigerator including one or more divided spaces including an input unit, a processor, and an output unit. The input unit is configured to recognize a wine label of each space and recognize an image for determining opening or non-opening of a wine. The processor is configured to acquire wine information by using an artificial intelligence model that receives image data acquired from the input unit as an input value, create a wine list table of each space by using the acquired information, and group wines having the same storage condition into at least one group according to the wine list table, and perform a control such that a temperature of each space is set based on the storage condition of the group. The output unit is configured to output a signal received from the processor.

US 2018/210418 A1 refers to an electronic device for food management and a control method thereof. The control method of an electronic device for food management includes measuring intensity of light, which is irradiated with food and reflected, by wavelengths using an infrared sensor; obtaining state information of the food based on the measured light intensity information; and adjusting at least one of temperature and humidity of an area where the food is disposed in the electronic device based on at least one of the obtained state information and preset usage plan information of the food.

Summary of the Invention

Technical Problem

The freshness control of perishable products is, for example, different by each perishable product, or changed in accordance with initial freshness at the time when a perishable product is contained in the container. For this reason, though the environment is controlled under the same uniform control conditions, it is difficult to perform proper freshness control.

An object of the present disclosure is to perform reinforcement learning on control conditions of the perishable product environment by using information regarding freshness of perishable products obtained by a freshness sensor to automatically control the perishable product environment.

Solution to Problem

A containing device of the present disclosure is a perishable product containing device according to independent claim 1.

This makes it possible to perform reinforcement learning on control conditions of the inside environment by using the information regarding the freshness of the perishable product obtained from the storage container to automatically control the perishable product environment.

Here, the learning unit may learn the inside environment for the freshness of the perishable product measured by the freshness measurement unit to maximize the reward decided by the reward decision unit, and the adjustment unit may adjust the inside environment of the storage container to cause the environmental information acquired by the environmental information acquisition unit to serve as the inside environment for the freshness learned by the learning unit.

This makes it possible to learn the control of the inside environment optimized with respect to the decrease in the freshness of the perishable product.

In addition, when inside environments before and after the certain period of time are different, the reward decision unit may decide the reward based on an inside environment at one or more points during the certain period of time.

This makes it possible to learn the environmental control in response to the change in the inside environment over a certain period of time.

In addition, the inside environment at one or more points during the certain period of time may include the inside environment at an end point of the certain period of time.

This makes it possible to learn the environmental control assuming the inside environment when the certain period of time has elapsed.

A method of operating the perishable product containing device of the invention is defined in claim 5. Moreover, the computer program of the present disclosure is defined in claim 6.

Accordingly, the reinforcement learning can be performed on the control conditions of the perishable product environment by using the information regarding the freshness of the perishable product to automatically control the perishable product environment.

Brief Description of Drawings

FIG. 1 is a diagram showing an overall configuration of a perishable product control system to which the present invention is applied;

FIG. 2 is a diagram showing a functional configuration example of an information processing device;

FIG. 3 is a diagram showing a hardware configuration example of the information processing device;

FIG. 4 is a diagram showing an example of an inside environment (inside temperature) over a certain period of time adjusted by an environment adjustment device;

FIG. 5 is a diagram showing another example of the inside environment (inside temperature) over a certain period of time adjusted by the environment adjustment device;

FIG. 6 is a diagram showing still another example of the inside environment (inside temperature) over a certain period of time adjusted by the environment adjustment device; and

FIG. 7 is a diagram showing still another example of the inside environment (inside temperature) over a certain period of time adjusted by the environment adjustment device.

Description of the invention

Hereinafter, an embodiment will be described in detail with reference to attached drawings.

<System configuration>

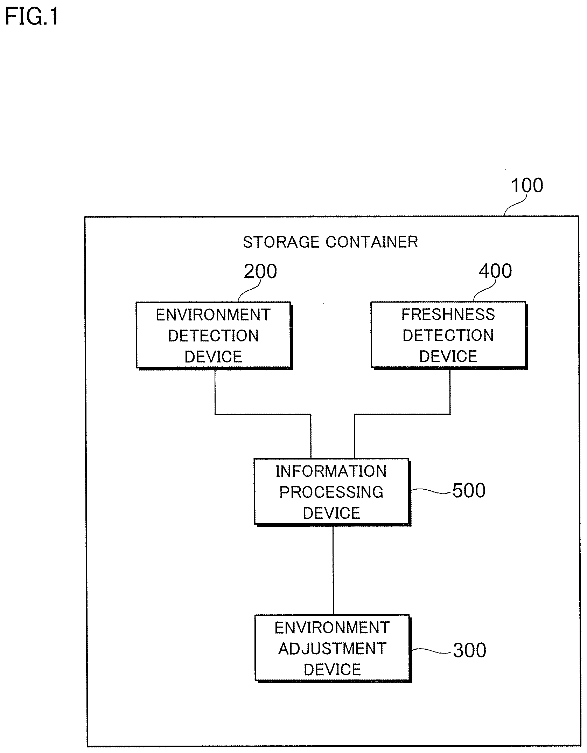

FIG. 1 is a diagram showing an overall configuration of a perishable product control system to which the present invention is applied. The perishable product control system of the present embodiment includes a storage container 100, an environment detection device 200, an environment adjustment device 300, a freshness detection device 400, and an information processing device 500.

The storage container 100 is a device that contains and stores perishable products. The storage container 100 has a delivery entrance (not shown) for carrying in the perishable products to be contained. By closing the delivery entrance of the storage container 100, inside space is sealed and separated from the external environment. The inside of the storage container 100 may be divided into multiple rooms. The storage container 100 is a container used for, for example, transporting the perishable products. As the storage container 100, those containing the perishable products in a refrigerated environment, those containing the perishable products in a frozen environment, those containing the perishable products in an ordinary-temperature environment, and so on can be assumed. The storage container 100 is an example of a containing device.

The environment detection device 200 is provided inside the storage container 100 and detects the environment inside the storage container 100 (the inside environment). The environment detection device 200 acquires data indicating the state of the internal space of the storage container 100 (hereinafter referred to as "environmental data"). Specifically, data indicating temperature, humidity, air pressure, the component of gas filling the internal space, etc., is acquired. Consequently, as the environment detection device 200, a temperature sensor, a humidity sensor, an air pressure sensor, sensors for various kinds of gas components, etc., can be used. In the case where the inside of the storage container 100 is divided into multiple rooms, the environmental data may be acquired individually from each room. The environment detection device 200 is an example of an environmental information acquisition unit.

The environment adjustment device 300 is provided in the storage container 100 and controls the inside environment of the storage container 100. Factors of the inside environment to be controlled by the environment adjustment device 300 correspond to factors of the inside environment to be detected by the environment detection device 200. Consequently. The environment adjustment device 300 controls the temperature, humidity, air pressure, components of gas filling the inside space, etc., to adjust the inside environment. In the case where the inside of the storage container 100 is divided into the multiple rooms, a different environment may be set for each room. The environment adjustment device 300 is an example of an adjustment unit.

The freshness detection device 400 detects the freshness of perishable products contained in the storage container 100. The freshness of a perishable product can be evaluated with various indexes. Specifically, "appearance (color, gloss)," "scent," "taste," "physical properties (hardness)," "water content," "ingredients (sugar, acid)," etc. are used as the freshness indexes. In addition, for meat and fish, the index called "K value" regarding the ratio of ingredients contained in these perishable products is generally used as an index representing the freshness. Consequently, as the freshness detection device 400, in accordance with the kinds of perishable products to be contained, a freshness sensor capable of acquiring data regarding these various indexes (hereinafter, referred to as "freshness data") is used.

The information processing device 500 processes data acquired by the environment detection device 200 and the freshness detection device 400, and controls the operation of the environment adjustment device 300 based on the obtained results. The information processing device 500 is implemented by, for example, a personal computer, a smartphone, an embedded computer in the storage container 100, etc. The information processing device 500 may be provided integrally with the storage container 100, or may be provided separately from the storage container 100 to acquire data from the environment detection device 200 and the freshness detection device 400 using a communication line. The configuration example in FIG. 1 shows the information processing device 500 provided integrally with the storage container 100. In the case where the information processing device 500 is provided separately from the storage container 100, the communication line for sending and receiving data may be a wired line or a wireless network.

<Configuration of information processing device 500>

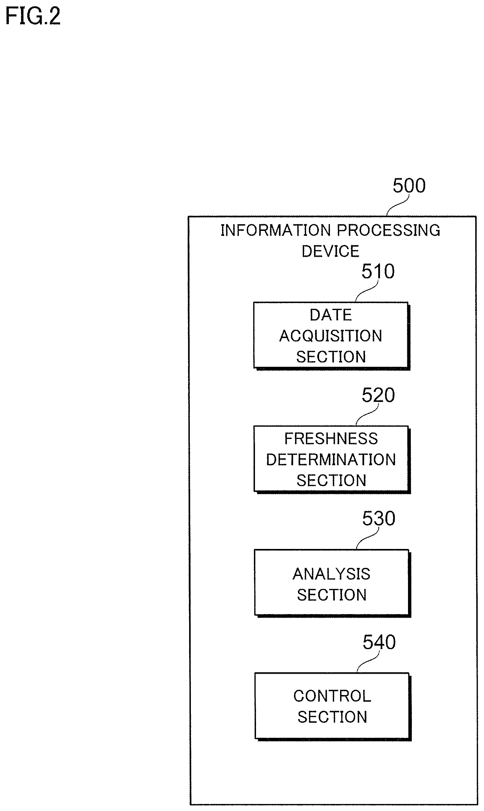

FIG. 2 is a diagram showing a functional configuration example of the information processing device 500. The information processing device 500 includes a data acquisition section 510, a freshness determination section 520, an analysis section 530, and a control section 540.

The data acquisition section 510 acquires data from the environment detection device 200 and the freshness detection device 400. Data is acquired, for example, at regular intervals. More specifically, environmental data is transmitted from the environment detection device 200 at regular intervals, which is acquired by the data acquisition section 510 of the information processing device 500. Freshness data is transmitted from the freshness detection device 400 at regular intervals, which is acquired by the data acquisition section 510 of the information processing device 500. The environmental data acquired from the environment detection device 200 is transmitted to the analysis section 530. The freshness data acquired from the freshness detection device 400 is transmitted to the freshness determination section 520.

The freshness determination section 520 determines the freshness of the perishable products contained in the storage container 100 based on the freshness data obtained by the freshness detection device 400. This provides information regarding the freshness of the perishable products. As described above, the freshness of the perishable products is determined based on the various freshness indexes. Therefore, the freshness determination section 520 determines the freshness by a method specified in accordance with the kind of perishable products contained in the storage container 100 and the type of freshness data obtained by the freshness detection device 400. The freshness may be determined using existing determination methods, for example, a method using the K values performed for fish and shellfish, or meat. The freshness determination section 520 is an example of a freshness information acquisition unit. In addition, the freshness detection device 400 and the freshness determination section 520 of the information processing device 500 are an example of a freshness measurement unit.

The analysis section 530 analyzes the information regarding the inside environment (the environmental data) acquired by the data acquisition section 510 and the freshness information of the perishable products determined by the freshness determination section 520, to thereby find the proper inside environment. The proper inside environment differs depending on the kinds and states of the perishable products contained in the storage container 100. It is needless to say that the proper inside environment differs depending on the kind of perishable product, but the proper inside environment is sometimes different for even the same kind of perishable product depending on the state thereof. For example, regarding tomatoes, the proper ranges of temperature and humidity are different between the fully ripened tomatoes and mature-green tomatoes. In addition, the fully ripened tomatoes produce a large amount of ethylene and have low ethylene sensitivity, whereas the mature-green tomatoes produce a small amount of ethylene and have high ethylene sensitivity. Therefore, the proper gas components are also different. In addition, the range of proper temperature for potatoes differs between the unripened state and the fully ripened state. Further, different freshness of the same kind of perishable product corresponds to different proper inside environment. For example, the state of high freshness, as compared to the state of low freshness, makes it possible to select the inside environment with the assumption of long-term storage. As described above, the analysis section 530 is required to identify the inside environment in consideration of various factors of the target perishable product.

In the present invention, the analysis section 530 uses a learning model obtained by the reinforcement learning to select the inside environment to be adjusted by the environment adjustment device 300. The learning model used by the analysis section 530 is obtained by performing reinforcement learning with settings of "state s" for the information regarding the inside environment and the freshness information, "action a" for the control condition of the environment adjustment device 300, and "reward r" for the degree of decrease in the freshness after elapse of a certain period of time. The "reward r" is set so that the smaller the decrease in the freshness, the larger the value of the "reward r." Thus, according to the learning model, based on the "state s" obtained by the environment detection device 200, the freshness detection device 400, and the freshness determination section 520, the "action a" is optimized to minimize the decrease in the freshness (to maximize the "reward r") after elapse of a certain period of time in the inside environment controlled by the "action a."

In addition, the analysis section 530 may actually control the environment adjustment device 300 to perform freshness control of the perishable products while adjusting the inside environment by the information processing device 500 provided in the storage container 100, to thereby proceed with the reinforcement learning using the results of the freshness control. Specifically, the above-described learning model may be updated (setting of the "reward r" may be changed) based on the information regarding the inside environment actually adjusted, and the state of decrease in the freshness of the perishable product after elapse of a certain period of time from the start of control by the environment adjustment device 300. The analysis section 530 is an example of a learning unit and a reward decision unit.

The control section 540 controls the operation of the environment adjustment device 300 by generating a control instruction for the environment adjustment device 300, and transmitting the generated control instruction to the environment adjustment device 300. The control section 540 controls the environment adjustment device 300 to have the inside environment selected by the analysis section 530. In other words, the control section 540 controls the environment adjustment device 300 so that the environmental information acquired by the environment detection device 200 is that of the inside environment for the freshness of the perishable product learned at the analysis section 530 (the inside environment selected by the learning model). The control of the inside environment is individually performed for each of environmental factors, such as temperature, humidity, air pressure, and gas component, by comparing the current inside environment with the inside environment selected by the analysis section 530. Focusing on the inside temperature as an example, if the current inside temperature is the same as the inside temperature selected by the analysis section 530, the control section 540 controls the environment adjustment device 300 to maintain the current inside temperature. In addition, in the case where the current inside temperature is different from the inside temperature selected by the analysis section 530, the control section 540 controls the environment adjustment device 300 so that the inside temperature becomes the latter temperature.

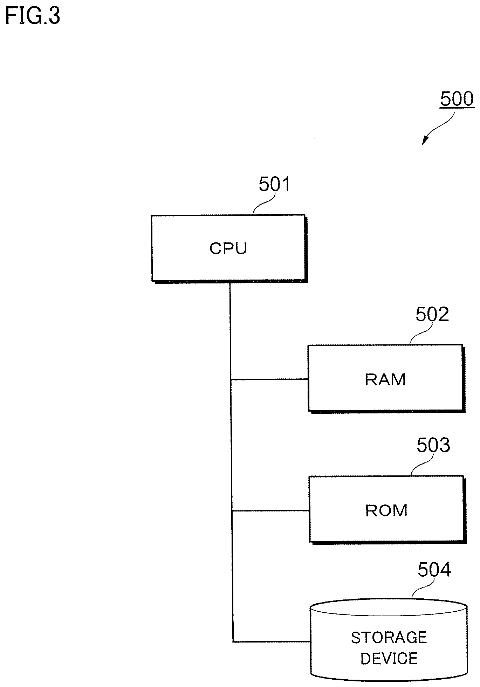

FIG. 3 is a diagram showing a hardware configuration example of the information processing device 500. The information processing device 500 is implemented by a computer. The computer that implements the information processing device 500 includes a CPU (Central Processing Unit) 501, which is an arithmetic unit, a RAM (Random Access Memory) 502, which is a storage unit, a ROM (Read Only Memory) 503, and a storage device 504. The RAM 502 is a main storage device (main memory), and used as a working memory when the CPU 501 performs arithmetic processing. The ROM 503 holds programs and data of setting values prepared in advance, etc., and the CPU 501 can execute processing by directly reading the programs and data from the ROM 503. The storage device 504 is a storing unit for programs and data. The storage device 504 stores the programs, and the CPU 501 reads the programs stored in the storage device 504 into the main storage device to execute thereof. In addition, the storage device 504 stores the processing results by the CPU 501 to preserve thereof. Moreover, the storage device 504 stores the learning model by the above-described reinforcement learning, which is used to select the inside environment. As the storage device 504, for example, a magnetic disk device, an SSD (Solid State Drive), etc. can be used.

In the case where the information processing device 500 is implemented by the computer shown in FIG. 3, each of the functions of the data acquisition section 510, the freshness determination section 420, the analysis section 530, and the control section 540, which have been described with reference to FIG. 2, is implemented by the CPU 501 executing the programs. The information processing device 500 implementing each of the above-described functions by execution of the programs by the CPU 501 is an example of a learning device.

<Learning example of inside environment adjustment>

As described above, the analysis section 530 of the information processing device 500 uses a learning model obtained by the reinforcement learning to select the inside environment to be adjusted by the environment adjustment device 300. It has been described that, as the learning model, the model obtained by performing reinforcement learning with settings of "state s" for the information regarding the inside environment and the freshness information, "action a" for the control condition of the inside environment, and "reward r" for the degree of decrease in the freshness after elapse of a certain period of time is used; the further description will be given of the "state s," the "action a," and the "reward r."

The inside environment is adjusted by the environment adjustment device 300 over a certain period of time (for example, 6 hours, 12 hours, 1 day, 3 days, 1 week, etc.). Consequently, for the inside environment as the "state s," the inside environment during the certain period of time is considered. The operation of the environment adjustment device 300 during the certain period of time is the target of the "action a." The "reward r" is determined based on the difference between the freshness at the start point and the freshness at the end point of the certain period of time.

The inside environment during the certain period of time will be considered further. If the inside environment is adjusted by the environment adjustment device 300 over a certain period of time, depending on the adjustment, the inside environment sometimes differs between the start point and the end point of the certain period of time. In addition, the operation of the environment adjustment device 300 during the certain period of time dynamically changes in some cases. In this case, even though the inside environment at the start point is the same, it is assumed that the degree of decrease in the freshness of the perishable product is different in response to the inside environment at the end point or midway through the period of time. Consequently, the "state s" is identified by considering the inside environment, not only at the start point, but also at the end point of and midway through the certain period of time. In addition, with regard to the control of the environment adjustment device 300 as the "action a," even though the inside environment at the start point is the same, multiple "action a" can be set with the inside environments at the end point of and midway through the certain period of time that are different. Hereinafter, specific description will be given of some examples. In the following examples, the inside temperature will be focused on as a specific example of the inside environment.

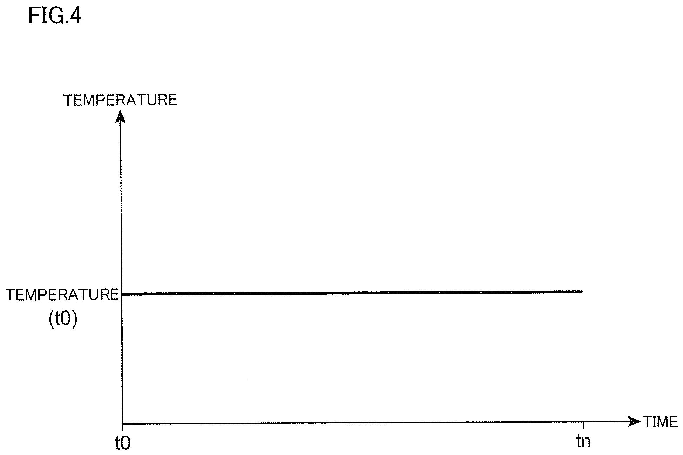

FIG. 4 is a diagram showing an example of the inside environment (inside temperature) over a certain period of time adjusted by the environment adjustment device 300. In the example shown in FIG. 4, the environment adjustment device 300 adjusts the inside temperature to be constant at the temperature (t0) during the period t(0-n) from the start point t0 to the end point tn. In this case, since the inside temperature is constant throughout the period t(0-n), the "state s" in the reinforcement learning is the temperature (t0). In addition, the operation of the environment adjustment device 300 as the "action a" is controlled so that the "state s" at the temperature (t0) continues for a certain period of time t(0-n). Then, the "reward r" is set based on the difference between the freshness of the perishable product at the point t0 and the freshness of the perishable product at the point tn.

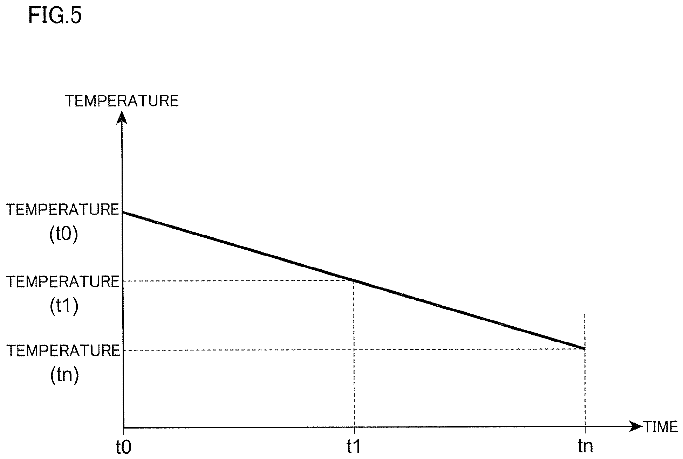

FIG. 5 is a diagram showing another example of the inside environment (inside temperature) over a certain period of time adjusted by the environment adjustment device 300. In the example shown in FIG. 5, the environment adjustment device 300 adjusts the inside temperature to change from the temperature (t0) to the temperature (tn) during the period t(0-n) from the start point t0 to the end point tn. Note that, in the example, the temperature change is adjusted to be constant with the elapsed time. In this case, the temperature (t0) at the start point t0 of the period of time t(0-n) and the temperature (tn) at the end point tn are different; accordingly, the temperature (t0) at the start point cannot simply be the "state s." Therefore, the temperature at a specific point within the period t(0-n) is set to the "state s." For example, it can be considered that a temperature (t1) at a middle point t1 between the point t0 and the point tn is set to the "state s." This means that the "action a" is considered as controlling the operation of the environment adjustment device 300 so that the inside temperature at the point t0 is the temperature (t0), and the inside temperature after elapse of the period of time t(0-n) reaches the temperature (tn), and the "state s" is considered as the inside temperature (t1) throughout the period of time t(0-n). Then, the "reward r" is set based on the difference in the freshness between the point t0 and the point tn. Here, in the example shown in FIG. 5, since the degree of temperature change is constant,

$$\text{period } t(0\text{-}1) = \text{period } t(0\text{-}n)/2,$$

and

$$\text{temperature } (t1) = \text{temperature } (t0) + (\text{temperature } (tn) - \text{temperature } (t0))/2$$

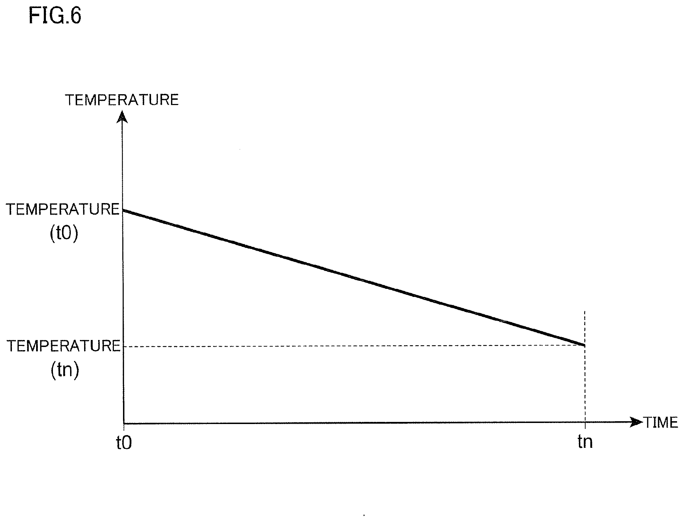

FIG. 6 is a diagram showing still another example of the inside environment (inside temperature) over a certain period of time adjusted by the environment adjustment device 300. In the example shown in FIG. 6, the environment adjustment device 300 adjusts the inside temperature to change from the temperature (t0) to the temperature (tn) during the period t(0-n) from the start point t0 to the end point tn. Note that, in the example, the temperature change is adjusted to be constant with the elapsed time. In this case, the temperature (t0) at the start point t0 of the period of time t(0-n) and the temperature (tn) at the end point tn are different; accordingly, the temperature (t0) at the start point cannot simply be the "state s." The above is similar to the example described with reference to FIG. 5; however, in this example, both the temperature (t0) at the start point t0 and the temperature (tn) at the end point tn of the period t(0-n) are set as the "state s." The "action a" is the operation control of the environment adjustment device 300, which implements such temperature changes. Then, the "reward r" is set based on the difference in the freshness between the point t0 and the point tn.

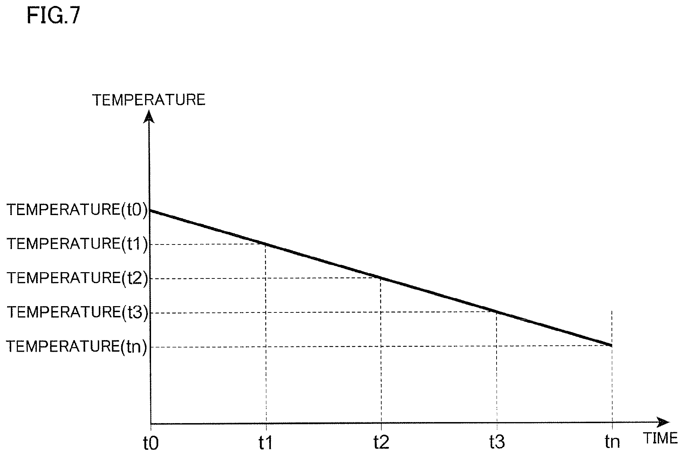

FIG. 7 is a diagram showing still another example of the inside environment (inside temperature) over a certain period of time adjusted by the environment adjustment device 300. In the example shown in FIG. 7, the environment adjustment device 300 adjusts the inside temperature to change from the temperature (t0) to the temperature (tn) during the period t(0-n) from the start point t0 to the end point tn. Note that, in FIG. 7, the temperature change is constant with the elapsed time, but this example does not specify how the temperature changes. For example, the temperature may be changed rapidly during the first half of the period t(0-n), and may be changed slowly during the second half. In addition, the temperature may be changed several times in a stepwise manner. In the example shown in FIG. 7, one or more points are set within the period t(0-n), and the "state s" is set based on the temperature at each point. For example, a representative value resulting from the statistical treatment of the temperature at each point may be set to the "state s." An average value, a median value, etc. can be selected as the representative value, but the representative value may be set in accordance with how the temperature changes during the period t(0-n).

In the example shown in FIG. 7, three points, from the point t1 to the point t3, are set between the start point t0 and the end point tn, to thereby obtain five temperatures, the temperature (t0), the temperature (t1), the temperature (t2), the temperature (t3), and the temperature (tn). These points and temperatures may be set in accordance with how the temperature changes during the period of time t(0-n). Then, the representative value (for example, the average value) of these five temperatures is given as the "state s." In addition, the operation control of the environment adjustment device 300 to achieve the above-described temperature change during the period t(0-n) is given as the "action a." Then, the "reward r" is set based on the difference in the freshness between the point t0 and the point tn. Note that, in this example, the environment adjustment device 300 starts the control from the current inside environment to obtain the inside environment at the point when the period t(0-n) has elapsed (the point tn); accordingly, the point to obtain the inside environment may surely include the point tn.

Note that, in the examples described with reference to FIGS. 4 to 7, the description has been given of the case in which the inside temperature is controlled as one of the inside environments, but it is possible to similarly analyze each factor of the inside environment, such as humidity or gas component, that can be detected by the environment detection device 200 and adjusted by the environment adjustment device 300. In addition, each factor of the inside environment may be weighted, or conditions may be added in accordance with the kind and state of the perishable products, the freshness of the perishable products at the time of analysis, etc. For example, since the storable period in the storage container 100 differs depending on the types of perishable products, different periods can be set to the period t(0-n) shown in the above-described analysis example.

So far, the embodiment has been described, but the technical scope of the present disclosure is not limited to the above-described embodiment. For example, in the above-described embodiment, the information processing device 500 has been described as the device that implements each of the function to process the data acquired by the environment detection device 200 and the freshness detection device 400, the function to control the operation of the environment adjustment device 300 based on the results of the processing, and the function to perform the reinforcement learning based on the acquired data; however, these functions may be implemented by individual pieces of hardware. The device for performing the learning function is also implemented as a learning device that learns by use of data acquired and collected by the environment detection devices 200 and the freshness detection devices 400 of the multiple storage containers 100.

In addition, the above-described embodiment has shown that the analysis section 530 of the information processing device 500 learns to obtain a proper inside environment in accordance with the kinds and states of the perishable products. Consequently, when the freshness of the perishable products is controlled, the user identifies the perishable products to be controlled on the information processing device 500, and uses the learning model corresponding to the identified perishable products to control the freshness. In contrast thereto, a means of identifying the kinds of perishable products subjected to the freshness control may be provided in the information processing device 500. This makes it possible for the information processing device 500 to identify the perishable products to be controlled and perform the freshness control using the corresponding learning model when the freshness of the perishable products is controlled. The perishable products may be identified, for example, by image analysis using images obtained by photographing the perishable products.

Here, the above-described embodiment can be viewed as follows. The learning device of the present disclosure includes: a freshness determination section 520 acquiring information regarding freshness of a perishable product contained in a storage container; and an analysis section 530 learning an inside environment of the storage container for the freshness of the perishable product acquired by the freshness determination section 520 to decide a reward used in the learning. The analysis section 530 is a learning device of the inside environment for perishable products that decides the reward based on the decrease in the freshness over a certain period of time under the inside environment for the freshness determined based on the freshness acquired by the freshness determination section 520, and learns the inside environment for the freshness based on the decided reward.

In this manner, the reinforcement learning can be performed on control conditions of the perishable product environment by using the information regarding the freshness of perishable products to automatically control the perishable product environment.

Here, in the case where the inside environments before and after the certain period of time are constant, the analysis section 530 may decide the reward based on the inside environment at the start point of the certain period of time.

In this manner, the environmental control to provide the constant inside environment over a certain period of time can be learned.

Alternatively, in the case where the inside environment before and after the certain period of time is different, the analysis section 530 may decide the reward based on the inside environment at a specific point during the certain period of time.

In this manner, in the case where the inside environment changes during the certain period of time, the environmental control based on the inside environment at the specific point can be learned.

Alternatively, in the case where the inside environment before and after the certain period of time is different, the analysis section 530 may decide the reward based on the inside environment at the start point and the inside environment at the end point of the certain period of time.

In this manner, in the case where the inside environment changes during the certain period of time, the environmental control based on the inside environments at the start point and the end point of the certain period of time can be learned.

Alternatively, in the case where the inside environments before and after the certain period of time are different, the analysis section 530 may find the representative value of environmental information values indicating the inside environment at multiple points during the certain period of time, and may decide the reward based on the representative value.

In this manner, the environmental control in response to the change in the inside environment over a certain period of time can be learned.

In addition, the above-described embodiment can also be viewed as follows. The containing device of the present disclosure includes: the storage container 100 containing perishable products; the environment adjustment device 300 adjusting the inside environment including at least the temperature inside the storage container 100; the environment detection device 200 acquiring the environmental information including at least the temperature inside the storage container 100; the freshness detection device 400 and the freshness determination section 520 measuring the freshness of the perishable products contained in the storage container 100; and the analysis section 530 learning the inside environment of the storage container 100 for the freshness of the perishable products measured by the freshness detection device 400 and the freshness determination section 520 to decide the reward. Based on the freshness measured by the freshness detection device 400 and the freshness determination section 520, the environmental information acquired by the environment detection device 200, and the learning results of the analysis section 530, the environment adjustment device 300 operates to cause the inside environment of the storage container 100 to serve as the inside environment for the freshness. The analysis section 530 is the perishable product containing device that decides the reward based on the decrease in the freshness over a certain period of time under the inside environment adjusted by the environment adjustment device 300, and learns the inside environment for the freshness based on the decided reward.

In this manner, the reinforcement learning can be performed on control conditions of the inside environment by using the information regarding the freshness of perishable products obtained from the storage container 100 to automatically control the perishable product environment.

Here, the analysis section 530 may learn the inside environment for the freshness of perishable products measured by the freshness detection device 400 and the freshness determination section 520 to maximize the reward decided by the analysis section 530, and the environment adjustment device 300 may adjust the inside environment of the storage container 100 to cause the environmental information acquired by the environment detection device 200 to serve as the inside environment for the freshness learned by the analysis section 530.

In this manner, it is possible to learn the control of the inside environment optimized with respect to decrease in the freshness of perishable products.

In addition, in the case where the inside environments before and after the certain period of time are different, the analysis section 530 may decide the reward based on the inside environment at one or more points during the certain period of time.

In this manner, the environmental control in response to the change in the inside environment over a certain period of time can be learned.

Moreover, the inside environments at one or more points during the certain period of time may include the inside environment at the end point of the certain period of time.

In this manner, it is possible to learn the environmental control assuming the inside environment when the certain period of time has elapsed.

Reference Signs List

100 Storage container
200 Environment detection device
300 Environment adjustment device
400 Freshness detection device
500 Information processing device
510 Data acquisition section
520 Freshness determination section
530 Analysis section
540 Control section

**Claims**

1.  A perishable product containing device comprising:

    a storage container (100) containing a perishable product;
    an adjustment unit (300) adjusting an inside environment of the storage container, the inside environment including at least a temperature;
    an environmental information acquisition unit (200) acquiring environmental information inside the container, the environmental information including at least the temperature;
    a freshness measurement unit (400, 520) measuring freshness of the perishable product contained in the storage container;

    **characterized in that** the perishable product containing device further comprises: a learning unit (530) learning by reinforcement learning the control of the inside environment of the storage container to minimize the decrease in the freshness of the perishable product, measured by the freshness measurement unit; and

    a reward decision unit (530) deciding a reward (r) used by the learning unit, wherein,
    based on the freshness measured by the freshness measurement unit and the environmental information acquired by the environmental information acquisition unit as state (s) of the reinforcement learning model, and a learning result of the learning unit, the adjustment unit operates to cause the inside environment of the storage container to minimize the decrease in the freshness of the perishable product, being a control condition of the adjustment unit used as action (a) of the reinforcement learning model,
    the reward decision unit decides the reward (r) of the reinforcement learning model based on a decrease in the freshness over a certain period of time under the inside environment adjusted by the adjustment unit, and
    the learning unit learns the inside environment for the freshness based on the reward (r) decided by the reward decision unit.

2.  The perishable product containing device according to claim 1, wherein

    the learning unit learns the inside environment for the freshness of the perishable product measured by the freshness measurement unit to maximize the reward (r) decided by the reward decision unit, and
    the adjustment unit adjusts the inside environment of the storage container to cause the environmental information acquired by the environmental information acquisition unit to serve as the inside environment for the freshness learned by the learning unit.

3.  The perishable product containing device according to claim 2, wherein, when inside environments before and after the certain period of time are different, the reward decision unit decides the reward (r) based on an inside environment at one or more points during the certain period of time.

4.  The perishable product containing device according to claim 3, wherein the inside environment at one or more points during the certain period of time includes an inside environment at an end point of the certain period of time.

5.  Method of operating a perishable product containing device according to any one of the preceding claims, the method comprising the steps of:

    acquiring information regarding freshness of a perishable product contained in the storage container;
    learning by reinforcement learning the control of the inside environment of the storage container to minimize the decrease in the acquired freshness of the perishable product;
deciding a reward (r) used in the reinforcement learning model, and
adjusting the inside environment of the storage container to minimize the decrease in the freshness of the perishable product, based on the freshness measured by the freshness measurement unit and the environmental information acquired by the environmental information acquisition unit used as state (s) of the reinforcement learning model, and a learning result of the learning unit,
wherein,
the control condition of the step of adjusting the inside environment of the storage container is used as action (a) of the reinforcement learning model,
in the step of deciding the reward (r), the reward (r) is decided based on a decrease in the freshness over a certain period of time under the inside environment for the freshness, the inside environment being determined based on the freshness of the perishable product acquired by the step of acquiring the information regarding the freshness, and,
in the step of learning, the inside environment for the freshness is learned based on the reward (r) decided by the step of deciding the reward (r).

6. A computer program comprising instructions to cause the perishable product containing device of any of claims 1 to 4 to carry out the steps of the method of claim 5.

**Patentansprüche**

1. Speichervorrichtung für verderbliche Produkte, umfassend:

einen Speicherbehälter (100), der ein verderbliches Produkt enthält;
eine Anpassungseinheit (300), die eine Innenumgebung des Speicherbehälters anpasst, wobei die Innenumgebung mindestens eine Temperatur einschließt;
eine Umgebungsinformationserfassungseinheit (200), die Umgebungsinformationen innerhalb des Behälters erfasst, wobei die Umgebungsinformationen mindestens die Temperatur einschließen;
eine Frischemesseinheit (400, 520), die Frische des im Speicherbehälter enthaltenen verderblichen Produkts misst;
**dadurch gekennzeichnet, dass** die Speichervorrichtung für verderbliche Produkte weiter Folgendes umfasst:

eine Lerneinheit (530), die durch Verstärkungslernen die Steuerung der Innenumgebung des Speicherbehälters lernt, um den durch die Frischemesseinheit gemessenen Rückgang von Frische des verderblichen Produkts zu minimieren; und
eine Belohnungsentscheidungseinheit (530), die eine durch die Lerneinheit verwendete Belohnung (r) entscheidet, wobei,
basierend auf der durch die Frischemesseinheit gemessenen Frische und die durch die Umgebungsinformationserfassungseinheit als Zustand (s) des Verstärkungslernmodells erfassten Umgebungsinformationen und einem Lernergebnis der Lerneinheit die Anpassungseinheit betrieben wird, um zu bewirken, dass die Innenumgebung des Speicherbehälters den Rückgang von Frische des verderblichen Produkts minimiert, was eine Steuerbedingung der Anpassungseinheit ist, die als Aktion (a) des Verstärkungslernmodells verwendet wird,
die Belohnungsentscheidungseinheit die Belohnung (r) des Verstärkungslernmodells basierend auf einem Rückgang von Frische über eine bestimmte Zeitdauer unter der durch die Anpassungseinheit angepasste Innenumgebung entscheidet, und
die Lerneinheit die Innenumgebung für die Frische basierend auf der durch die Belohnungsentscheidungseinheit entschiedene Belohnung (r) lernt.

2. Speichervorrichtung für verderbliche Produkte nach Anspruch 1, wobei

die Lerneinheit die Innenumgebung für die durch die Frischemesseinheit gemessene Frische des verderblichen Produkts lernt, um die durch die Belohnungsentscheidungseinheit entschiedene Belohnung (r) zu maximieren, und
die Anpassungseinheit die Innenumgebung des Speicherbehälters anpasst, um zu bewirken, dass die durch die Umgebungsinformationserfassungseinheit erfassten Umgebungsinformationen als die Innenumgebung für die durch die Lerneinheit gelernte Frische dienen.

3. Speichervorrichtung für verderbliche Produkte nach Anspruch 2, wobei, wenn die Innenumgebung vor und nach der bestimmten Zeitdauer unterschiedlich ist, die Belohnungsentscheidungseinheit die Belohnung (r) basierend auf einer Innenumgebung an einem oder mehreren Punkten während der bestimmten Zeitdauer entscheidet.

4. Speichervorrichtung für verderbliche Produkte nach Anspruch 3, wobei die Innenumgebung an einem oder mehreren Punkten während der bestimmten Zeitdauer eine Innenumgebung an einem Endpunkt der bestimmten Zeitdauer einschließt.

5. Verfahren zum Betrieb einer Speichervorrichtung für verderbliche Produkte nach einem der vorstehenden Ansprüche, wobei das Verfahren folgende Schritte umfasst:

Erfassen von Informationen in Bezug auf Frische eines im Speicherbehälter enthaltenen verderblichen Produkts;
Erlernen durch Verstärkungslernen der Steuerung der Innenumgebung des Speicherbehälters, um den Rückgang der erfassten Frische des verderblichen Produkts zu minimieren;
Entscheiden einer Belohnung (r), die in dem Verstärkungslernmodell verwendet wird, und
Anpassen der Innenumgebung des Speicherbehälters, um den Rückgang von Frische des verderblichen Produkts zu minimieren, basierend auf der durch die Frischemesseinheit gemessenen Frische und den durch die Umgebungsinformationserfassungseinheit erfassten Umgebungsinformationen, die als Zustand (s) des Verstärkungslernmodells verwendet werden, und einem Lernergebnis der Lerneinheit,
wobei,
die Steuerbedingung des Schritts des Anpassens der Innenumgebung des Speicherbehälters als Aktion (a) des Verstärkungslernmodells verwendet wird,
im Schritt des Entscheidens der Belohnung (r) die Belohnung (r) basierend auf einem Rückgang von Frische über eine bestimmte Zeitdauer unter der Innenumgebung für die Frische entschieden wird, wobei die Innenumgebung basierend auf der Frische des verderblichen Produkts bestimmt wird, die durch den Schritt des Erfassens der Informationen in Bezug auf die Frische erfasst wird, und,
im Schritt des Lernens die Innenumgebung für die Frische basierend auf der durch den Schritt des Entscheidens der Belohnung (r) entschiedenen Belohnung (r) gelernt wird.

6. Computerprogramm, umfassend Anweisungen, um zu bewirken, dass die Speichervorrichtung für verderbliche Produkte nach einem der Ansprüche 1 bis 4 die Schritte des Verfahrens nach Anspruch 5 ausführt.

**Revendications**

1. Dispositif contenant un produit périssable comprenant :

un conteneur de stockage (100) contenant un produit périssable ;
une unité d'ajustement (300) ajustant un environnement intérieur du conteneur de stockage, l'environnement intérieur incluant au moins une température ;
une unité (200) d'acquisition d'informations environnementales acquérant des informations environnementales à l'intérieur du conteneur, les informations environnementales incluant au moins la température ;
une unité (400, 520) de mesure de fraîcheur mesurant la fraîcheur du produit périssable contenu dans le conteneur de stockage ;

**caractérisé en ce que** le dispositif contenant un produit périssable comprend en outre :

une unité d'apprentissage (530) apprenant par apprentissage par renforcement à réguler l'environnement intérieur du conteneur de stockage afin de réduire au minimum la perte de la fraîcheur du produit périssable, mesurée par l'unité de mesure de fraîcheur ; et
une unité (530) de décision de récompense décidant d'une récompense (r) utilisée par l'unité d'apprentissage, dans lequel, sur la base de la fraîcheur mesurée par l'unité de mesure de fraîcheur et des informations environnementales acquises par l'unité d'acquisition d'informations environnementales en tant qu'état (s) du modèle d'apprentissage par renforcement, et d'un résultat d'apprentissage de l'unité d'apprentissage, l'unité d'ajustement fonctionne de façon à amener l'environnement intérieur du conteneur de stockage à réduire au minimum la perte de la fraîcheur du produit périssable, étant une condition de régulation de l'unité d'ajustement utilisée comme action (a) du modèle d'apprentissage par renforcement,
l'unité de décision de récompense décide de la récompense (r) du modèle d'apprentissage par renforcement sur la base d'une perte de la fraîcheur sur une période de temps donnée au sein de l'environnement intérieur ajusté par l'unité d'ajustement, et

l'unité d'apprentissage apprend l'environnement intérieur en tenant compte de la fraîcheur sur la base de la récompense (r) décidée par l'unité de décision de récompense.

2. Dispositif contenant un produit périssable selon la revendication 1, dans lequel

l'unité d'apprentissage apprend l'environnement intérieur en tenant compte de la fraîcheur du produit périssable mesurée par l'unité de mesure de fraîcheur pour optimiser la récompense (r) décidée par l'unité de décision de récompense, et

l'unité d'ajustement ajuste l'environnement intérieur du conteneur de stockage pour amener les informations environnementales acquises par l'unité d'acquisition d'informations environnementales à servir d'environnement intérieur en tenant compte de la fraîcheur apprise par l'unité d'apprentissage.

3. Dispositif contenant un produit périssable selon la revendication 2, dans lequel, lorsque les environnements intérieurs avant et après la période de temps donnée sont différents, l'unité de décision de récompense décide de la récompense (r) sur la base d'un environnement intérieur à un ou plusieurs instants pendant la période de temps donnée.

4. Dispositif contenant un produit périssable selon la revendication 3, dans lequel l'environnement intérieur à un ou plusieurs instants pendant la période de temps donnée inclut un environnement intérieur à un instant de fin de la période de temps donnée.

5. Procédé pour faire fonctionner un dispositif contenant un produit périssable selon l'une quelconque des revendications précédentes, le procédé comprenant les étapes suivantes :

acquisition d'informations concernant la fraîcheur d'un produit périssable contenu dans le conteneur de stockage ;

apprentissage par apprentissage par renforcement de la régulation de l'environnement intérieur du conteneur de stockage afin de réduire au minimum la perte de la fraîcheur acquise du produit périssable ;

décision d'une récompense (r) utilisée dans le modèle d'apprentissage par renforcement, et

ajustement de l'environnement intérieur du conteneur de stockage pour réduire au minimum la perte de la fraîcheur du produit périssable, sur la base de la fraîcheur mesurée par l'unité de mesure de fraîcheur et des informations environnementales acquises par l'unité d'acquisition d'informations environnementales utilisées en tant qu'état (s) du modèle d'apprentissage par renforcement, et d'un résultat d'apprentissage de l'unité d'apprentissage,

dans lequel,

la condition de régulation de l'étape d'ajustement de l'environnement intérieur du conteneur de stockage est utilisée comme action (a) du modèle d'apprentissage par renforcement,

dans l'étape de décision de la récompense (r), la récompense (r) est décidée sur la base d'une perte de la fraîcheur sur une période de temps donnée au sein de l'environnement intérieur en tenant compte de la fraîcheur, l'environnement intérieur étant déterminé sur la base de la fraîcheur du produit périssable acquise par l'étape d'acquisition des informations concernant la fraîcheur, et,

dans l'étape d'apprentissage, l'environnement intérieur en tenant compte de la fraîcheur est appris sur la base de la récompense (r) décidée par l'étape de décision de la récompense (r).

6. Programme informatique comprenant des instructions pour amener le dispositif contenant un produit périssable selon l'une quelconque des revendications 1 à 4 à mettre en œuvre les étapes du procédé selon la revendication 5.

FIG.1

100

STORAGE CONTAINER

200

ENVIRONMENT
DETECTION
DEVICE

400

FRESHNESS
DETECTION
DEVICE

INFORMATION
PROCESSING
DEVICE

500

ENVIRONMENT
ADJUSTMENT
DEVICE

300

FIG.2

500

INFORMATION PROCESSING
DEVICE

510

DATE
ACQUISITION
SECTION

520

FRESHNESS
DETERMINATION
SECTION

530

ANALYSIS
SECTION

540

CONTROL
SECTION

FIG.3

FIG.4

FIG.5

FIG.6

FIG.7

**EP 4 130 621 B1**

REFERENCES CITED IN THE DESCRIPTION

**Patent documents cited in the description**

- JP 2019041601 A
- WO 2018017450 A1
- WO 2018017817 A1
- US 2020012247 A1
- US 2018210418 A1